(19) Europäisches Patentamt
European Patent Office
Office européen des brevets

(11) **EP 4 016 082 B1**

(12) **EUROPÄISCHE PATENTSCHRIFT**

(45) Veröffentlichungstag und Bekanntmachung des Hinweises auf die Patenterteilung:
**22.02.2023  Patentblatt 2023/08**

(21) Anmeldenummer: **21195352.6**

(22) Anmeldetag: **07.09.2021**

(51) Internationale Patentklassifikation (IPC):
**G01N 33/58** (2006.01)     **G01N 33/564** (2006.01)
**G06K 9/62** (2006.01)       **G06V 10/44** (2022.01)
**G06V 20/69** (2022.01)      **G06T 7/10** (2017.01)
**G06T 7/00** (2006.01)       **G06N 3/02** (2006.01)
**G06N 3/08** (2006.01)       **G06T 7/11** (2017.01)

(52) Gemeinsame Patentklassifikation (CPC):
**G01N 33/582; G01N 33/564; G06F 18/2414;
G06N 3/084; G06T 7/0012; G06T 7/11;
G06V 10/44; G06V 20/695; G06V 20/698;**
G06T 2207/10024; G06T 2207/10056;
G06T 2207/10064; G06T 2207/20081;
G06T 2207/30024

(54) **VERFAHREN UND VORRICHTUNG ZUM DETEKTIEREN EINER PRÄSENZ EINES FLUORESZENZMUSTERTYPS AUF EINEM ORGANSCHNITT MITTELS IMMUNFLUORESZENZMIKROSKOPIE**

METHOD AND DEVICE FOR DETECTING A PRESENCE OF A FLUORESCENCE PATTERN TYPE ON AN ORGAN SEGMENT BY MEANS OF IMMUNOFLUORESCENCE MICROSCOPY

PROCÉDÉ ET DISPOSITIF DE DÉTECTION D'UNE PRÉSENCE D'UN TYPE DE MOTIF FLUORESCENT SUR UNE SECTION D'ORGANE PAR MICROSCOPIE IMMUNOFLUORESCENTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2020  EP 20215995**

(43) Veröffentlichungstag der Anmeldung:
**22.06.2022  Patentblatt 2022/25**

(73) Patentinhaber: **Euroimmun Medizinische Labordiagnostika AG**
**23560 Lübeck (DE)**

(72) Erfinder:
• **GERLACH, Stefan**
  **23560, Lübeck (DE)**
• **KRAUTH, Jens**
  **23560, Lübeck (DE)**
• **MARZAHL, Christian**
  **23560, Lübeck (DE)**
• **KRAUSE, Christian**
  **23560, Lübeck (DE)**
• **HOCKE, Jens**
  **23560, Lübeck (DE)**
• **DANCKWARDT, Maick**
  **23560, Lübeck (DE)**
• **HAHN, Melanie**
  **23560, Lübeck (DE)**
• **VOIGT, Jörn**
  **23560, Lübeck (DE)**

(56) Entgegenhaltungen:
**EP-A1- 3 258 247     EP-A1- 3 712 618
EP-A1- 3 767 587**

**Beschreibung**

Die Erfindung betrifft ein Verfahren sowie eine Vorrichtung zum Detektieren einer potentiellen Präsenz eines Fluoreszenzmustertyps auf einem Organschnitt mittels Immunfluoreszenzmikroskopie und mittels digitaler Bildverarbeitung.

EP3258247 A1 enthüllt ein Verfahren zum Detektieren wenigstens einer potentiellen

Präsenz wenigstens eines Fluoreszenzmustertyps auf einem Organschnitt mittels Immunfluoreszenzmikroskopie aber kein Verfahren, bei dem neuronale Netzwerke zum Einsatz kommen,

EP3712618 A1 enthüllt ein Verfahren, bei dem neuronale Netze Teilflächen eines Fluoreszenzbildes bestimmen, welche für eine Ausbildung des Fluoreszenzbildes relevant sind, wobei Parasiten untersucht werden.

EP3767587 A1 enthüllt ein Verfahren, in dem ein "Zellsubstrat" verwendet wird, welches humane Epithel- oder Epitheliomzellen (z.B. HEP-2-Zellen) aufweist zum Nachweis von primären Antikörpern in flüssigen Patientenproben.

Eine Immunfluoreszenzmikroskopie bzw. indirekte Immunfluoreszenzmikroskopie ist ein invitro-Test für eine Bestimmung einer Präsenz humaner Antikörper gegen bestimmte Antigene, um eine diagnostische Fragestellung beantworten bzw. einschätzen zu können. Derartige Antigene sind beispielsweise in bestimmten Bereichen von Organschnitten wie eines Rattenmagens vorhanden. Als Substrat dient also ein Organschnitt, welcher mit einer Patientenprobe in Form von Blut oder verdünntem Blut oder aber Blutserum oder verdünntem Blutserum inkubiert wird. Die Patientenprobe weist also potentiell bestimmte primäre Antikörper auf, welche Ausdruck eines Vorhandenseins einer Erkrankung des Patienten sein können. Solche primären bzw. spezifischen Antikörper können dann an Antigene des Substrats bzw. des Organschnittes binden. Derartig gebundene primäre Antikörper können dann dadurch markiert werden, dass in einem weiteren Inkubationsschritt sogenannte sekundäre Antikörper, vorzugsweise Anti-Human-Antikörper, an die gebundenen primären Antikörper anbinden und später dadurch sichtbar gemacht werden können, dass die sekundären Antikörper mit einem Fluoreszenzfarbstoff markiert sind. Ein solcher Fluoreszenzfarbstoff ist vorzugsweise ein grüner Fluoreszenzfarbstoff, insbesondere der Fluoreszenzfarbstoff FITC. Eine solche Bindung eines primären Antikörpers gemeinsam mit einem fluoreszenzmarkierten sekundären Antikörper kann dann später dadurch sichtbar gemacht werden, dass der Organschnitt mit Anregungslicht einer bestimmten Wellenlänge bestrahlt wird und somit die gebundenen Fluoreszenzfarbstoffe zur Emission von Fluoreszenzstrahlung angeregt werden.

Abhängig von der diagnostischen Fragestellung kann auf eine Präsenz eines oder ganz bestimmter Fluoreszenzmustertypen auf bestimmten Organschnitten bzw. ganz bestimmten Teilbereichen bzw. Teilflächen der Organschnitte abgestellt werden. Es ergibt sich also die Aufgabe, mittels digitaler Bildverarbeitung im Zuge einer Immunfluoreszenzmikroskopie bei einem in der vorgeschriebenen Weise inkubierten Organschnitt mittels digitaler Bildverarbeitung einen oder mehrere Fluoreszenzmustertypen in einem Fluoreszenzbild einer Immunfluoreszenzmikroskopie zu Detektieren.

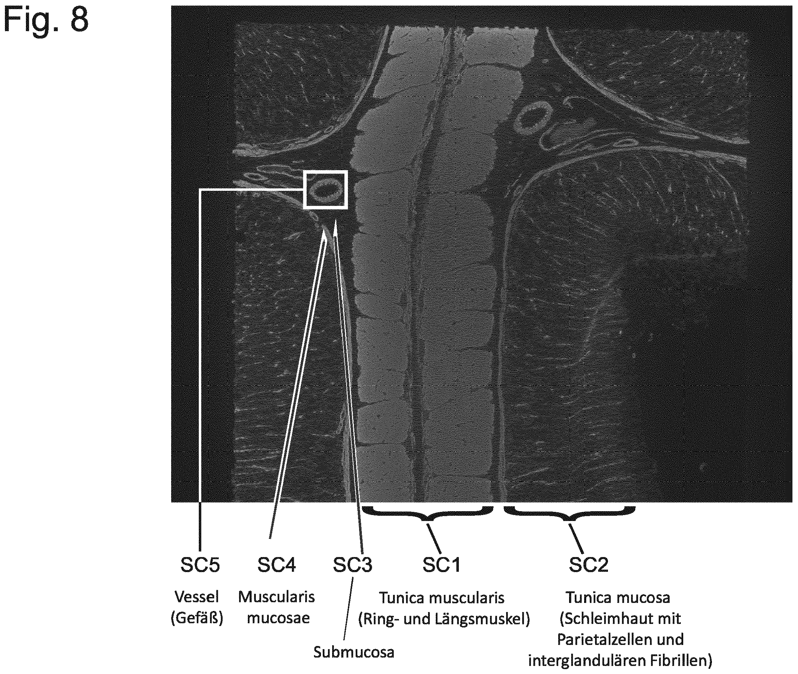

Die Fig. 8 zeigt ein Fluoreszenzbild eines Magens einer Ratte als ein Beispiel eines Organschnittes. Ein solcher Organschnitt weist unterschiedliche Organschichten auf. Organschichten sind hierbei beispielsweise die sogenannte Schleimschicht SC2 bzw. die Schleimhaut mit Parietalzellen und interglandulären kontraktilen Fibrillen, auch Tunica mucosa genannt.

Eine weitere Organschicht ist beispielsweise der Ring- und Längsmuskel, auch Tunica muscularis SC1 genannt. Eine weitere Organschicht ist beispielsweise die sogenannte Verschiebeschicht, auch Submucosa SC3 genannt. Eine wiederum andere Organschicht ist beispielsweise die Muscularis mucosae SC4. Eine wiederum andere Organschicht sind beispielsweise die Gefäße SC5.

Für ein beispielhaftes Anti-Smooth-Muscle-Antibody-Muster (ASMA) sind für eine Detektion des Musters zwei bestimmte Organschichten relevant: zum einen Tunica muscularis und zum anderen Tunica mucosa. Mittels Detektion einer Präsenz des ASMA-Musters kann dann möglicherweise anschließend durch einen Arzt oder Experten auf Vorliegen einer Hepatitis-Erkrankung geschlossen werden.

Eine Präsenz eines sogenannten ASMA-Musters zeigt sich insofern, dass es auf den zwei zuvor genannten Organschichten zu jeweiligen, bestimmten Teil-Fluoreszenzmustern kommt, welche gemeinsam das Fluoreszenzmuster ASMA bilden. Insbesondere wird hier auf eine Kombination aus einem netz- oder gitterartigen Muster in der Organschicht Tunica muscularis sowie auch ein Muster feiner Linien (interglanduläre kontraktile Fibrillen) auf der Tunica-mucosa-Organschicht abgestellt. Es ist daher notwendig, dass in dem Fluoreszenzbild diese beiden zuvor genannten Organschichten zu einem ausreichenden Grad bzw. mit einer ausreichenden Teilfläche bezogen auf die Gesamtfläche des Fluoreszenzbildes vorhanden sind, damit eine Präsenz des ASMA-Musters mittels einer digitalen Bildverarbeitung sicher detektiert werden kann.

Die Erfinder haben also erkannt, dass bei dem Prinzip der Immunfluoreszenzmikroskopie basierend auf Organschnitten in der Produktion bestimmte negative Auswirkungen vorkommen können, welche einer sicheren Detektion einer Präsenz eines Fluoreszenzmusters durch Bildverarbeitung entgegenwirken können. Ein Organschnitt wie aus der Fig. 8 weist möglicherweise die beiden zuvor genannten Organschichten jeweils nicht zu einem hinreichenden Flächenanteil auf. Durch Produktionsfehler kann es dazu kommen, dass zumindest eine der beiden Schichten oder aber beide Schichten jeweils nur zu einem sehr geringen Flächenanteil in dem Fluoreszenzbild vorhanden sind. Eine Detektion des Fluoreszenzmusters mittels digitaler Bildverarbeitung und neuronaler Netze kann dann möglicherweise zu Fehlergebnissen führen, welches vermieden werden sollte.

Im Produktionsprozess steht notwendiges Organmaterial nicht unbegrenzt zur Verfügung. Ein größerer Organschnitt wird zunächst auf eine Trägerfläche aufgebracht und dann die Trägerfläche in Teilträgerflächen auf Glas zerteilt, vorzugsweise mittels Schneiden, so dass es gerade in gewissen Bereichen des Organschnittes nur zu einer teilweisen Abdeckung des Objektträgers kommen kann. Daher kann im Zuge der Produktion es möglich sein, dass bestimmte Organschichten nur zu einem geringen Anteil in dem Organschnitt vorhanden sind.

Alternativ oder zusätzlich kann ein weiterer negativer technischer Effekt auftreten: Zur hinreichenden Darstellung bzw. Erkennbarkeit von Mustern in einem Fluoreszenzbild werden mitunter die Fluoreszenzbilder unter Verwendung von Mikroskopoptiken bestimmter optischer Vergrößerung erfasst. Dies kann zu einem Fluoreszenzbild führen, welches nicht den gesamten Objektträger und auch nicht den gesamten Organschnitt erfasst bzw. darstellt. Auch hierdurch kann es dazu kommen, dass eine bestimmte Organschicht nur zu einem geringen Anteil in dem Fluoreszenzbild des Organschnittes vorhanden ist.

Vorgeschlagen wird daher ein erfindungsgemäßes Verfahren zum Detektieren einer potentiellen Präsenz eines Fluoreszenzmustertyps auf einem Organschnitt mittels Immunfluoreszenzmikroskopie und mittels digitaler Bildverarbeitung.

Das Verfahren weist verschiedene Schritte auf. Zunächst erfolgt ein Bereitstellen des Organschnittes auf einem Objektträger. Anschließend erfolgt ein Inkubieren des Organschnittes mit einer flüssigen Patientenprobe, welche potentiell primäre Antikörper aufweist. Ferner erfolgt ein Inkubieren des Organschnittes mit sekundären Antikörpern, welche mit einem Fluoreszenzfarbstoff markiert sind. Es erfolgt dann ein Erfassen eines Fluoreszenzbildes des Organschnittes in einem Farbkanal, welcher zu dem Fluoreszenzfarbstoff korrespondiert. Ferner erfolgt ein Bereitstellen des Fluoreszenzbildes an ein neuronales Netz.

Das Verfahren zeichnet sich dadurch aus, dass mittels eines neuronalen Netzes gleichzeitig eine Segmentierungsinformation durch Segmentieren des Fluoreszenzbildes bestimmt wird und dass ferner eben auch gleichzeitig ein Konfidenzmaß, welches eine tatsächliche Präsenz des Fluoreszenzmustertyps indiziert, bestimmt wird.

Ferner erfolgt ein Bestimmen wenigstens einer Teilfläche des Fluoreszenzbildes, welche für eine Ausbildung des Fluoreszenzmustertyps relevant ist, auf Basis der zuvor bestimmten Segmentierungsinformation.

Ferner erfolgt ein Bestimmen einer Validitätsinformation, welche einen Grad einer Validität des Konfidenzmaßes indiziert, auf Basis der zuvor bestimmten wenigstens einen Teilfläche.

Ferner erfolgt schließlich ein Ausgeben des Konfidenzmaßes der tatsächlichen Präsenz des Fluoreszenzmustertyps in Abhängigkeit der Validitätsinformation.

Zur Verdeutlichung eines oder mehrerer möglicher Vorteile erfolgt nun im Weiteren eine genauere Darlegung verschiedener Aspekte des erfindungsgemäßen Verfahrens.

Wie bereits zuvor ausgeführt, können unterschiedliche Probleme bei der Anfertigung von Organschnitten auf Objektträgern auftreten, so dass für ein zu detektierendes Muster bzw. Fluoreszenzmuster relevante Organschichten nicht zu einem hinreichenden Abdeckungsgrad bzw. hinreichender Teilflächen vorhanden sind. Dadurch, dass das erfindungsgemäße Verfahren überprüft, ob eine bestimmte Organschicht als eine Teilfläche, welche für eine Ausbildung des Fluoreszenzmustertyps relevant ist, zu einem hinreichenden Grad präsent ist, und dass dann die Validitätsinformation auf Basis der zuvor bestimmten Teilfläche ermittelt wird, kann das Ausgeben des Konfidenzmaßes entsprechend gesteuert bzw. beeinflusst werden. Mit anderen Worten: Das Konfidenzmaß kann anhand der Teilfläche überprüft werden. Eine bestimmte Teilfläche korrespondiert also zu einer bestimmten Organschicht. Die Teilfläche des Fluoreszenzbildes ist also insbesondere eine Teilfläche, welche einem bestimmten Organabschnitt bzw. einer bestimmten Organschicht auf Basis der Segmentierungsinformation zugeordnet wird. Mit anderen Worten: Die Teilfläche wird als eine Teilfläche bestimmt, welche eine bestimmte Organschicht repräsentiert, wobei diese Teilfläche bzw. diese Organschicht auf Basis der Segmentierungsinformation bestimmt wird.

Es kann also somit durch Überprüfung der Teilfläche gewährleistet werden, dass das ermittelte Konfidenzmaß bezüglich der Präsenz des Fluoreszenzmustertypen auch valide ist, da in dem Fall, dass beispielsweise die Teilfläche bzw. die Organschicht eine zu geringe Größe bzw. ein zu geringes Maß aufweist, das Konfidenzmaß als nicht valide detektiert werden kann.

Es kann beispielsweise in dem Fall, dass die Teilfläche zu klein ist, das Konfidenzmaß vorzugsweise nicht ausgegeben werden.

Ferner ist das vorgeschlagene Verfahren insbesondere aus einem weiteren Grund vorteilhaft. Das eine neuronale Netz bestimmt gleichzeitig die Segmentierungsinformation bezogen auf das Fluoreszenzbild sowie das Konfidenzmaß für die Präsenz des Fluoreszenzmustertypen. Das neuronale Netz ist also so konzipiert, dass gleichzeitig sowohl Informationen über das Fluoreszenzmuster als auch wenigstens einer bestimmten sichtbaren Organschicht bzw. deren Teilfläche in die Analyse durch das neuronale Netz in die Bestimmung des Konfidenzmaßes bezüglich der Präsenz des Fluoreszenzmustertyps einfließen können. Mit anderen Worten: Das neuronale Netz ist ein vortrainiertes neuronales Netz, welches während des Trainings sowohl Konfidenzmaße hinsichtlich eines Vorliegens bzw. einer Präsenz des Fluoreszenzmustertypen als auch eine Segmentierungsinformation bezogen auf ein Segmentieren des Fluoreszenzbildes erlernt hat. Hierbei repräsentiert die Segmentierungsinformation insbesondere mehrere Teilsegmentierungsinformationen, welche jeweils für sich jeweilige unterschiedliche Organschichten des Organschnittes repräsentieren.

Es erfolgt eben erfindungsgemäß gerade nicht eine Bildverarbeitung, welche aus dem Stand der Technik bekannt ist: Hierbei können im Stand der Technik zunächst sogenannte Masken in Form von Bildsegmenten bzw. als Segmentierungsinformationen ermittelt werden und dann über das eigentliche Fluoreszenzbild gelegt werden, bevor ein neuronales Netz dann nur noch jene maskierten Teilbereiche des Fluoreszenzbildes analysiert, welche durch die Maske bzw. die Segmentierungsinformation herausgefiltert wurden, um ein Konfidenzmaß zu bestimmen. Hierbei würde also zunächst die Segmentierungsinformation final ermittelt werden, dann anschließend als Maske auf das Fluoreszenzbild angewendet werden und nur noch mittels Maskierung ermittelter Teilbildbereiche des Fluoreszenzbildes Eingang in die Analyse bzw. das Bestimmen der Konfidenzmaße hinsichtlich der Präsenz des Fluoreszenzmusters finden.

Ein solches Verfahren nach dem Stand der Technik wird gerade erfindungsgemäß nicht verfolgt, da eben gleichzeitig die Segmentierungsinformation als auch das Konfidenzmaß durch das neuronale Netz ermittelt werden. Insbesondere erfolgt vorzugsweise in dem erfindungsgemäßen Verfahren zunächst eine Transformation des Fluoreszenzbildes in den sogenannten Feature-raum bzw. Merkmalsraum durch Transformation des Fluoreszenzbildes mittels wenigstens einer Convolutional-Operation, wobei dann erst nach dieser Transformation in den Merkmalsraum die sich ergebenden Feature-Informationen weiterverarbeitet werden, um auf Basis dieser Feature-Informationen sowohl die Segmentierungsinformation als auch das Konfidenzmaß zu ermitteln. In diesem vorzugsweise ausgestalteten Verfahren gemäß einer bevorzugten Ausführungsform wird dann also eben nicht die Segmentierungsinformation bzw. Segmentierungsmasken über das Fluoreszenzbild gelegt, sondern in der Verarbeitung des neuronalen Netzes bedingen sich die Bestimmung der Segmentierungsinformation und die Bestimmung des Konfidenzmaßes gegenseitig. Hierdurch kann vorzugsweise gerade in einer Trainingsphase des neuronalen Netzes die Bestimmung des Konfidenzmaßes dadurch vorteilhaft beeinflusst werden, dass die ebenfalls gleichzeitig bestimmte Segmentierungsinformation die Bestimmung des Konfidenzmaßes beeinflusst und somit implizit bestimmte Segmentierungsinformation bzw. bestimmte Organschichten hervorgehoben bzw. stärker Eingang finden können. Ein weiterer Vorteil besteht insbesondere darin, dass das Training des neuronalen Netzes nicht in zwei separaten Schritten stattfindet, sondern dass das Training gleichzeitig eine Optimierung des neuronalen Netzes hinsichtlich der Segmentierung als auch der Bestimmung des Konfidenzmaßes herbeiführt.

Vorteilhafte Ausführungsformen der Erfindung sind Gegenstand der abhängigen Ansprüche und werden in der folgenden Beschreibung unter teilweiser Bezugnahme auf die Figuren näher erläutert.

Vorzugsweise weist das Verfahren weitere Schritte auf: Bestimmen mehrerer Teilflächen des Fluoreszenzbildes, welche für eine Ausbildung des Fluoreszenzmustertyps relevant sind, auf Basis der Segmentierungsinformation, sowie Bestimmen einer Validitätsinformation, welche einen Grad einer Validität des Konfidenzmaßes indiziert, auf Basis der zuvor bestimmten Teilflächen.

Vorzugsweise weist das Verfahren weitere Schritte auf: Bestimmen eines Flächenanteils der wenigstens einen Teilfläche bezogen auf die Fläche des Fluoreszenzbildes sowie Bestimmen der Validitätsinformation auf Basis des Flächenanteils.

Vorzugsweise weist das Verfahren weitere Schritte auf: Bestimmen jeweiliger Flächenanteile der jeweiligen Teilfläche bezogen auf die Fläche des Fluoreszenzbildes sowie Bestimmen der Validitätsinformation auf Basis der Flächenanteile.

Vorzugsweise weist das Verfahren weitere Schritte auf: Bestimmen mehrerer Teilflächen des Fluoreszenzbildes, welche für eine Ausbildung des Fluoreszenzmustertyps relevant sind, auf Basis der Segmentierungsinformation, Bestimmen jeweiliger Flächenanteile der jeweiligen Teilflächen bezogen auf die Fläche des Fluoreszenzbildes, Bestimmen der Validitätsinformation auf Basis der Flächenanteile und auf Basis jeweiliger Schwellenwerte, Ausgeben des Konfidenzmaßes der tatsächlichen Präsenz des Fluoreszenzmustertyps in dem Fall, dass die jeweiligen Flächenanteile einen jeweiligen Schwellenwert überschreiten.

Vorzugsweise ist das neuronale Netz so ausgebildet, dass es zunächst auf Basis des Fluoreszenzbildes eine erste Menge mehrerer Feature-Informationen in einem Merkmalsraum mittels wenigstens einer oder mehrerer Convolutional-Operationen generiert und anschließend auf Basis der ersten Menge an Feature-Informationen die Segmentierungsinformation und das Konfidenzmaß bestimmt.

Vorzugsweise ist das neuronale Netz so ausgebildet, dass es zunächst auf Basis des Fluoreszenzbildes eine erste Menge mehrerer Feature-Informationen in einem Merkmalsraum mittels einer oder mehrerer Convolutional-Operationen generiert, anschließend auf Basis der ersten Menge an Feature-Informationen die Segmentierungsinformation bestimmt und anschließend auf Basis der ersten Menge an Feature-Informationen und auf Basis der Segmentierungsinformation das Konfidenzmaß bestimmt.

Vorzugsweise ist das neuronale Netz so ausgebildet, dass es zunächst auf Basis des Fluoreszenzbildes eine erste Menge mehrerer Feature-Informationen in einem Merkmalsraum mittels einer oder mehrerer Convolutional-Operationen generiert, anschließend auf Basis der ersten Menge an Feature-Informationen die Segmentierungsinformation bestimmt, anschließend auf Basis der Segmentierungsinformation eine zweite Menge mehrerer Feature-Informationen in einem Merkmalsraum mittels wenigstens einer Convolutional-Operation generiert und anschließend auf Basis der ersten Menge an Feature-Informationen und der zweiten Menge an Feature-Informationen das Konfidenzmaß bestimmt.

Vorzugsweise weist das Verfahren weitere Schritte auf: Bestimmen mehrerer Teilflächen des Fluoreszenzbildes, welche für eine Ausbildung des Fluoreszenzmustertyps relevant sind, auf Basis der Segmentierungsinformation, und in dem Fall, dass der Fluoreszenzmustertyp als tatsächlich präsent bestimmt wird, Bestimmen eines Helligkeitsgrades einer der Teilfläche in dem Fluoreszenzbild, welche für eine Ausbildung des Fluoreszenzmustertyps potentiell relevant ist, sowie Abschätzen eines maximalen Verdünnungsgrades der Patientenprobe, bei welchem eine Inkubation des Organschnittes mit der Patientenprobe noch zu einer Präsenz eines des Fluoreszenzmustertyps führt.

Vorgeschlagen wird ferner eine Vorrichtung zum Detektieren wenigstens einer potentiellen Präsenz wenigstens eines Fluoreszenzmustertyps auf einem Organschnitt mittels Immunfluoreszenzmikroskopie und mittels digitaler Bildverarbeitung, aufweisend eine Haltevorrichtung für einen Objektträger mit einem Organschnitt, welcher mit einer Patientenprobe, aufweisend die Autoantikörper, sowie ferner mit sekundären Antikörpern, welche jeweils mit einem Fluoreszenzfarbstoff markiert sind, inkubiert wurde, wenigstens eine Bilderfassungseinheit zum Erfassen eines Fluoreszenzbildes des Organschnittes in einem Farbkanal, welcher zu dem Fluoreszenzfarbstoff korrespondiert. Die Vorrichtung weist ferner wenigstens eine Recheneinheit auf, welche ausgebildet ist, das Fluoreszenzbild an ein neuronales Netz bereitzustellen, mittels des einen neuronalen Netzes gleichzeitig eine Segmentierungsinformation durch Segmentieren des Fluoreszenzbildes zu bestimmen und ferner ein Konfidenzmaß, welches eine tatsächliche Präsenz des Fluoreszenzmustertyps indiziert, zu bestimmen, wenigstens eine Teilfläche des Fluoreszenzbildes zu bestimmen, welche für eine Ausbildung des Fluoreszenzmustertyps relevant ist, auf Basis der Segmentierungsinformation, eine Validitätsinformation zu bestimmen, welche einen Grad einer Validität des Konfidenzmaßes (KM) indiziert, auf Basis der zuvor bestimmten wenigstens einen Teilfläche sowie das Konfidenzmaß der tatsächlichen Präsenz des Fluoreszenzmustertypes in Abhängigkeit der Validitätsinformation auszugeben.

Vorgeschlagen wird ferner ein Verfahren zur digitalen Bildverarbeitung, aufweisend die Schritte: Entgegennehmen eines Fluoreszenzbildes, welches eine Färbung eines Organschnittes, durch einen Fluoreszenzfarbstoff repräsentiert, Bereitstellen des Fluoreszenzbildes an ein neuronales Netz, gleichzeitiges Bestimmen einer Segmentierungsinformation durch Segmentieren des Fluoreszenzbildes und Bestimmen eines Konfidenzmaßes, welches eine tatsächliche Präsenz des Fluoreszenzmustertyps indiziert, mittels des einen gemeinsamen neuronalen Netzes, Bestimmen wenigstens einer Teilfläche des Fluoreszenzbildes, welche für eine Ausbildung des Fluoreszenzmustertyps relevant ist, auf Basis der Segmentierungsinformation, Bestimmen einer Validitätsinformation, welche einen Grad einer Validität des Konfidenzmaßes indiziert, auf Basis der zuvor bestimmten wenigstens einen Teilfläche, Ausgeben des Konfidenzmaßes der tatsächlichen Präsenz des Fluoreszenzmustertypes in Abhängigkeit der Validitätsinformation.

Vorgeschlagen wird ferner ein Computerprogrammprodukt, umfassend Befehle, die bei der Ausführung des Programms durch einen Computer diesen veranlassen, das Verfahren zur digitalen Bildverarbeitung durchzuführen.

Im Folgenden wird die Erfindung anhand spezieller Ausführungsformen ohne Beschränkung des allgemeinen Erfindungsgedankens anhand der Figuren näher erläutert. Dabei zeigen:

Fig. 1 eine Ausführungsform des erfindungsgemäßen Verfahrens,

Fig. 2 bevorzugte Schritte zum Bestimmen jeweiliger Flächenanteile,

Fig. 3 eine Überprüfung jeweiliger Teilflächen hinsichtlich jeweiliger Schwellenwerte,

Fig. 4 eine Ausführungsform des erfindungsgemäßen Verfahrens mit Bestimmungsschritten zur Generierung unterschiedlicher Mengen an Feature-Informationen,

Fig. 5 bevorzugte Schritte zur Abschätzung eines maximalen Verdünnungsgrades einer Patientenprobe, bei welchem eine Inkubation des Organschnittes mit der Patientenprobe noch zu einer Präsenz des Fluoreszenzmustertyps führt,

Fig. 6 bevorzugte Schritte eines vorgeschlagenen Verfahrens zur digitalen Bildverarbeitung,

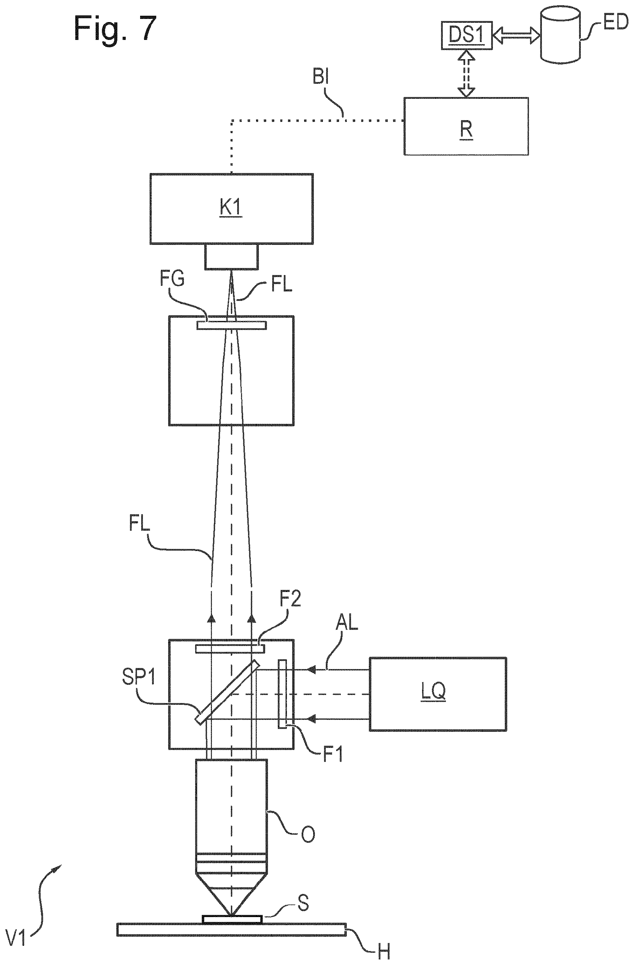

Fig. 7 eine Ausführungsform einer vorgeschlagenen Vorrichtung,

Fig. 8 einen beispielhaften Organschnitt mit unterschiedlichen Organschichten,

Fig. 9 ein Fluoreszenzbild wie aus Fig. 8 zusammen mit Segmentierungsinformation,

Fig. 10 unterschiedliche hervorgehobene Organschichten,

Fig. 11 Experimentalergebnisse,

Figur 12 eine Übersichtsstruktur einer Ausführungsform eines neuronalen Netzes sowie

Figur 13 eine detaillierte Darstellung einzelner Verarbeitungsblöcke des neuronalen Netzes.

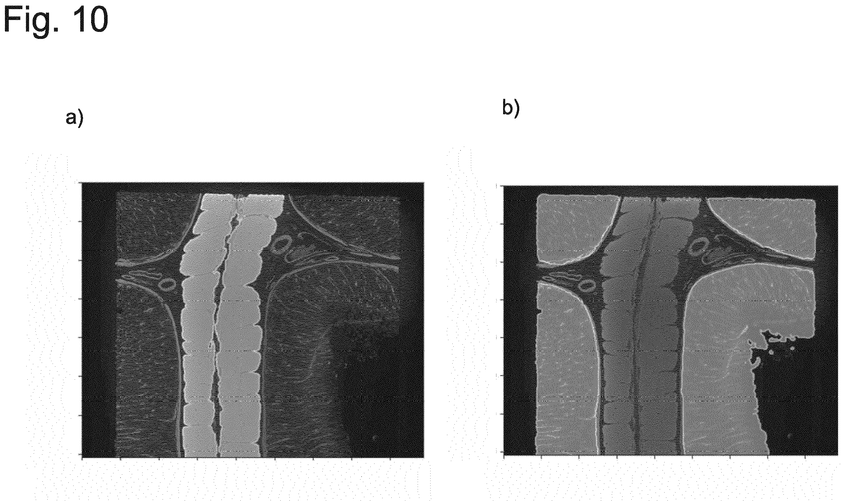

Figur 10 zeigt eine Vorrichtung V1, mittels welcher das erfindungsgemäße Verfahren vorzugsweise durchgeführt werden kann. Die Vorrichtung V1 kann als ein Fluoreszenzmikroskop bezeichnet werden. Die Vorrichtung V1 weist eine Halterung H für ein Substrat S bzw. einen Objektträger auf, welches bzw. welcher in der zuvor beschriebenen Weise inkubiert wurde. Über eine Optik O wird Anregungslicht AL einer Anregungslichtquelle LQ hin zu dem Substrat S geleitet. Sich ergebende Fluoreszenzstrahlung FL wird dann durch die Optik O zurücktransmittiert und passiert den dichroitischen Spiegel SP1 und einen optionalen optischen Filter F2. Vorzugsweise passiert die Fluoreszenzstrahlung FL einen optischen Filter FG, welcher einen Grünkanal herausfiltert. Eine Kamera K1 ist vorzugsweise eine Monochromkamera, welche dann bei Vorhandensein eines optischen Filters FG die Fluoreszenzstrahlung FL in einem Grünkanal erfasst. Gemäß einer alternativen Ausführungsform ist die Kamera K1 eine Farbbildkamera, welche ohne Verwendung des optischen Filters FG auskommt und mittels einer Bayer-Matrix das Fluoreszenzbild in dem entsprechenden Farbkanal als einen Grünkanal erfasst. Die Kamera K1 stellt die Bildinformation BI bzw. das Fluoreszenzbild an eine Recheneinheit R bereit, welche diese Bildinformation BI verarbeitet. Vorzugsweise kann die Recheneinheit R über eine Datenschnittstelle DS1 Daten ED wie beispielsweise ein Fluoreszenzbild, Konfidenzmaße und/oder eine Validitätsinformation ausgeben bzw. bereitstellen.

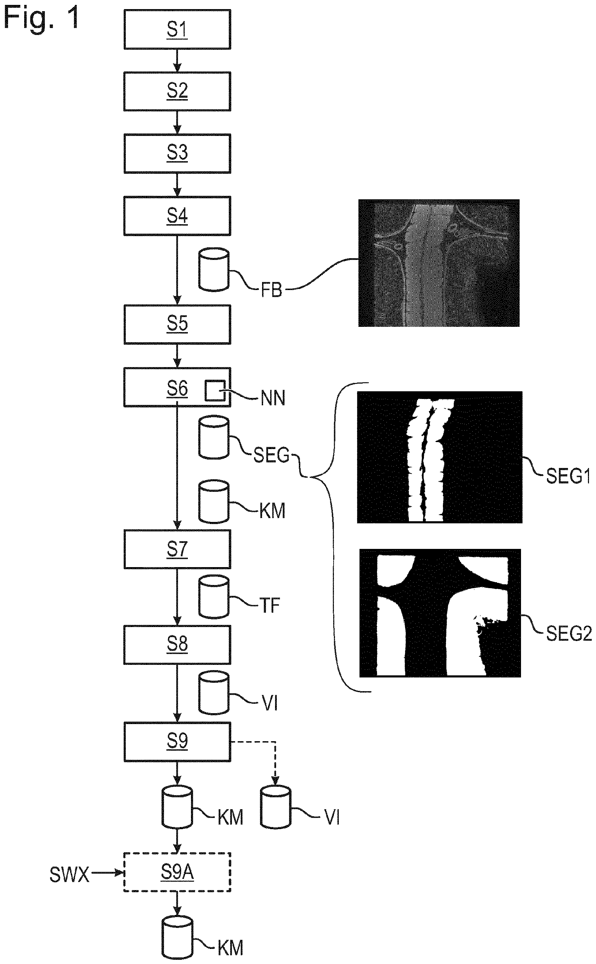

Die Fig. 1 zeigt Schritte einer Ausführungsform des vorgeschlagenen Verfahrens. In einem Schritt S1 wird der Organschnitt auf einem Objektträger bereitgestellt. In einem Schritt S2 erfolgt ein Inkubieren des Organschnittes mit einer flüssigen Patientenprobe, welche potentiell primäre Antikörper aufweist, In einem Schritt S3 erfolgt ein Inkubieren des Organschnittes mit sekundären Antikörpern, welche mit einem Fluoreszenzfarbstoff markiert sind.

In einem Schritt S4 erfolgt ein Erfassen eines Fluoreszenzbildes des Organschnittes in einem Farbkanal, welcher zu dem Fluoreszenzfarbstoff korrespondiert.

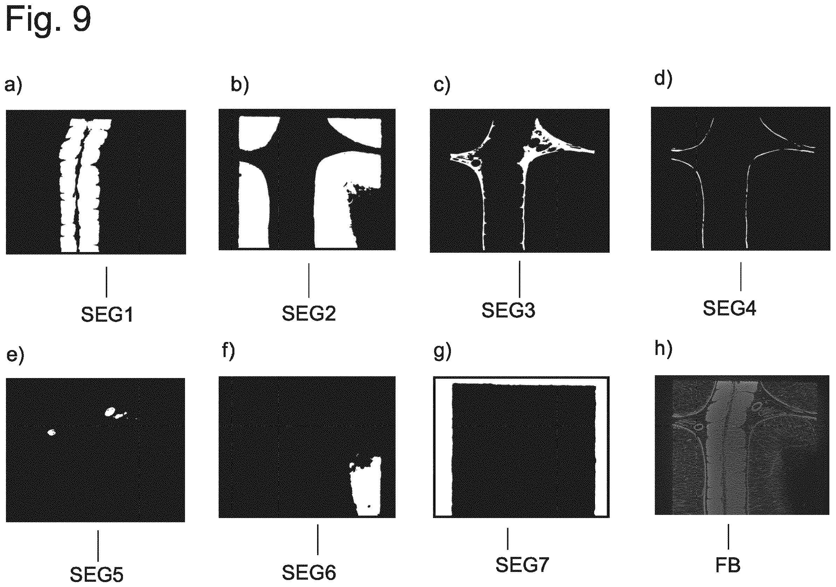

Es ergibt sich dann das Fluoreszenzbild FB, welches hier beispielsweise auch als ein Datenelement FB dargestellt ist. Ein solches Fluoreszenzbild ist auch beispielhaft in der Fig. 8 sowie der Fig. 9h dargestellt.

Die Fig. 8 illustriert für das Fluoreszenzbild FB die Organschicht Tunica muscularis bzw. als eine Schicht SC1, ferner die Organschicht Tunica mucosa als eine Schicht SC2, ferner die Schicht Submucosa als eine Schicht SC3, ferner die Schicht Muscularis mucosae als eine Schicht SC4 und ferner die Gefäß-Schicht als eine Schicht SC5.

Gemäß der Figur 1 erfolgt in einem Schritt S5 das Bereitstellen des Fluoreszenzbildes an ein neuronales Netz.

Das neuronale Netz kann beispielsweise als ein Netz NN in einem Schritt S6 verwendet werden. In dem Schritt S6 erfolgt gleichzeitig ein Bestimmen einer Segmentierungsinformation SEG durch Segmentieren des Fluoreszenzbildes FB. Diese Segmentierungsinformation SEG ist hier beispielhaft dargestellt als ein Datenelement SEG und kann beispielsweise zwei Teilsegmentierungsinformationen SEG1 und SEG2 aufweisen, welche auch in den Fig. 9a sowie 9b dargestellt sind. Ferner ermittelt das neuronale Netz gleichzeitig ein Konfidenzmaß KM bezüglich einer tatsächlichen Präsenz eines zu detektierenden Fluoreszenzmustertyps.

Vorzugsweise ermittelt das neuronale Netz NN nicht nur ein einzelnes Konfidenzmaß KM bezüglich eines einzelnen Fluoreszenzmustertyps bzw. einer einzelnen Präsenz eines einzelnen Fluoreszenzmustertyps, sondern das neuronale Netz NN ermittelt mehrere Konfidenzmaße bezüglich mehrerer Fluoreszenzmustertypen. In einem solchen Fall beinhaltet das Datenelement KM aus der Fig. 1, dargestellt jeweilige Konfidenzmaße für jeweilige Präsenzen jeweiliger Fluoreszenzmustertypen. Hierbei weist also beispielsweise das Datenelement KM aus der Fig. 1 nicht nur ein einzelnes Konfidenzmaß auf, sondern beispielsweise dreizehn Konfidenzmaße bezogen auf dreizehn unterschiedliche Fluoreszenzmustertypen. Eine solche vorzugsweise Ausgestaltung mit Bestimmung jeweiliger Konfidenzmaße jeweiliger tatsächlicher Präsenzen jeweiliger Fluoreszenzmustertypen ist insbesondere deshalb vorteilhaft, da dann bei einer Analyse des Fluoreszenzbildes FB durch das neuronale Netz NN ein Vorkommen unterschiedlicher Fluoreszenzmustertypen im Zuge der Lösung für möglich erachtet wird und eine genauere Abgrenzung bzw. Bestimmung des bestimmten Fluoreszenzmustertyps, dessen Präsenz ermittelt werden soll, im Zuge der Analyse durch das neuronale Netz berücksichtigt und ermöglicht wird. Es wird also hierbei nicht auf eine reine Positiv- oder Negativentscheidung bezüglich der Präsenz des bestimmten Fluoreszenzmustertypen, den man als präsent detektieren möchte, abgestellt, sondern andere mögliche Muster werden in dem Lösungsraum ebenfalls berücksichtigt.

Vorzugsweise bestimmt also das neuronale Netz jeweilige Konfidenzmaße hinsichtlich jeweiliger Präsenzen jeweiliger Fluoreszenzmustertypen, wobei ein bestimmtes Konfidenzmaß dieser Konfidenzmaße die tatsächliche Präsenz des bestimmten Fluoreszenzmustertypen indiziert. Es wird dann später im Zuge des Verfahrens vorzugsweise das eine bestimmte Konfidenzmaß der tatsächlichen Präsenz des bestimmten Fluoreszenzmustertypen in Abhängigkeit der Validitätsinformation ausgegeben.

Zur Fig. 1 kann ferner festgestellt werden, dass in einem Schritt S7 eine Teilfläche des Fluoreszenzbildes, welche für eine Ausbildung des Fluoreszenzmustertypen relevant ist, auf Basis der Segmentierungsinformation SEG bestimmt wird. Die Segmentierungsinformation SEG weist beispielsweise in einer bevorzugten Ausführungsform sieben verschiedene Teilsegmentierungsinformationen SEG1 bis SEG7 auf, wie in den Fig. 9a bis 9g dargestellt. Vorzugsweise können mehrere Segmentierungsinformationen bezüglich weiterer Schichten berücksichtigt werden. Beispielsweise kann eine Segmentierungsinformation bezüglich einem Vorliegen einer Organschicht der Magenhöhle, eine weitere Segmentierungsinformation bezüglich sogenannter Artefakte als auch wiederum eine weitere Segmentierungsinformation bezüglich anderer Organstrukturen vorgesehen werden. Es können beispielsweise bis zu elf verschiedene Segmentierungsinformationen vorgesehen werden.

Die Fig. 10a zeigt in einem Overlay in hervorgehobener Weise Muster der Organschicht Tunica muscularis basierend auf der zuvor ermittelten Segmentierungsinformation SEG1 aus der Fig. 9a. Die Fig. 10b zeigt in einem Overlay Musterausbildung in einem Bereich der Organschicht Tunica mucosae, wobei die Segmentierungsinformation SEG2 aus der Fig. 9b herangezogen wurde.

Für eine Präsenz eines Musters werden insbesondere nur solche Bildbereiche bzw. Teilflächen betrachtet bzw. herangezogen, welche für die Ausbildung des bestimmten Fluoreszenzmustertyps relevant sind. Dieses kann wenigstens eine Teilfläche des Fluoreszenzbildes sein, welche zu einer bestimmten entsprechenden Organschicht korrespondiert. Im Beispiel des ASMA-Musters werden beispielsweise mehrere Teilflächen des Fluoreszenzbildes bzw. mehrere Organschichten, beispielsweise zwei Organschichten, nämlich Tunica muscularis und Tunica mucosa, herangezogen bzw. betrachtet. Diese beiden Schichten sind für die Ausbildung des Fluoreszenzmustertyps ASMA relevant. Diese Bestimmung der Teilflächen des Fluoreszenzbildes bzw. der Teilflächen der entsprechenden Organschichten erfolgt auf Basis der entsprechenden Segmentierungsinformation, beispielsweise also in Segmentierungsinformation SEG1 aus Fig. 9a und SEG2 aus Fig. 9b. Die Teilfläche TF1 ist beispielsweise durch die weißen Pixel der Segmentierungsinformation SEG1 aus Fig. 9a gegeben. Die Teilfläche TF2 ist beispielsweise durch die weißen Pixel der Segmentierungsinformation SEG2 aus Fig. 9b gegeben.

Es erfolgt dann in einem Schritt S8 das Bestimmen der Validitätsinformation auf Basis der zuvor bestimmten wenigstens einen Teilfläche. In der Fig. 1 sind die eine oder mehrere Teilflächen als ein Datenelement TF dargestellt. Es erfolgt also das Bestimmen der Validitätsinformation auf Basis der vorbestimmten Teilfläche TF als eine Information VI, welche hier als ein Datenelement VI dargestellt ist.

Insbesondere erfolgt also ein Bestimmen der jeweiligen Flächenanteile der jeweiligen Teilflächen bzw. der jeweiligen Organschichten bezogen auf die Fläche des Fluoreszenzbildes und das Bestimmen der Validitätsinformation auf Basis der Flächenanteile.

Dies Validitätsinformation VI kann beispielsweise eine Boolesche Variable sein, welche den Wert 1 für den Fall annimmt, dass die zuvor mit Konfidenzmaß KM als valide angesehen werden.

In einem Schritt S9 wird dann in Abhängigkeit der Validitätsinformation VI jenes Konfidenzmaß KM ausgegeben, welches für die tatsächliche Präsenz des bestimmten Fluoreszenzmustertypen relevant ist.

Vorzugsweise kann in dem Fall, dass die Validitätsinformation VI eine Invalidität des Konfidenzmaßes KM indiziert, das Konfidenzmaß KM nicht ausgegeben werden. Insbesondere kann dann anstatt des Konfidenzmaßes KM eine Fehlermeldung ausgegeben werden, welche nicht explizit dargestellt ist.

Vorzugsweise wird in dem Schritt S9 auch die Validitätsinformation VI ausgegeben.

Das zuvor bestimmte Konfidenzmaß KM kann beispielsweise ein Vektor mehrerer Skalarwerte sein, wobei die jeweiligen Vektoreinträge jeweilige Konfidenzmaße bezogen auf jeweilige Fluoreszenzmustertypen repräsentieren bzw. indizieren. Vorzugsweise kann in dem Schritt S9 nur jener Skalarwert als Konfidenzmaß KM ausgegeben werden, welcher ein Konfidenzmaß bezogen auf das zu detektierende Fluoreszenzmuster, beispielsweise das ASMA-Muster, indiziert.

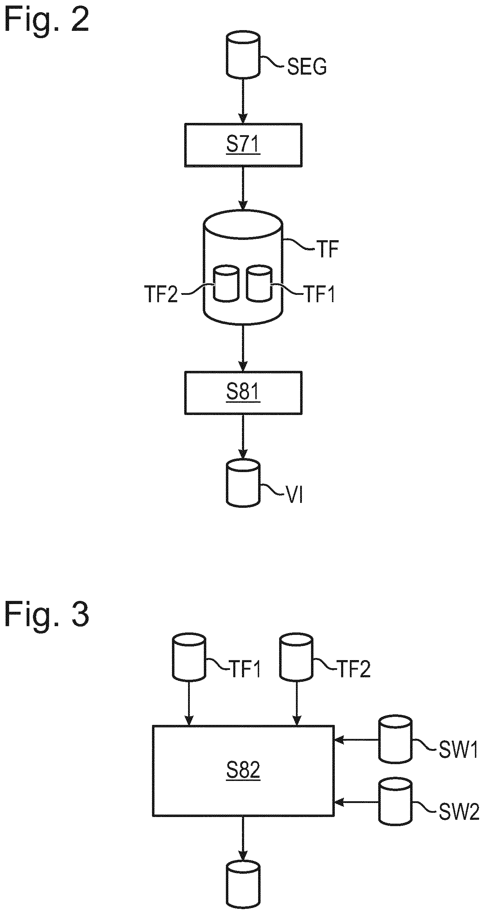

Die Fig. 2 zeigt einen zu dem Schritt S7 alternativen Schritt S71, in welchem auf Basis der Segmentierungsinformation SEG mehrere Teilflächen TF1, TF2, welche für eine Ausbildung des Fluoreszenzmustertyps relevant sind, bestimmt werden. Gerade im Beispiel des ASMA-Musters ergibt sich eine zuvor beschriebene Ausbildung des Musters über zwei unterschiedliche Organschichten, Tunica mucosae und Tunica muscularis, hinweg. Es müssen beide Organschichten zu einem hinreichenden Grad bzw. einem hinreichenden Flächenanteil in dem Fluoreszenzbild FB präsent sein.

Ferner zeigt die Fig. 2 ein zu dem Schritt S8 alternativen Schritt S81, in welchem dann die Validitätsinformation VI auf Basis der mehreren Teilflächen TF1, TF2 ermittelt wird.

Die Fig. 3 zeigt eine Überprüfung der Teilflächen TF1, TF2 anhand jeweiliger Schwellenwerte SW1, SW2.

Der hier dargestellte Schritt S82 ist ein Schritt, welcher alternativ zu dem Schritt S81 aus der Fig. 2 oder dem Schritt S8 aus der Fig. 1 durchgeführt werden kann. Es wird dann also die Validitätsinformation VI auf Basis der mehreren Teilflächen TF1, TF2 und der jeweiligen Schwellenwerte SW1, SW2 ermittelt. Die Teilflächen TF1 und TF2 weisen jeweilige Flächenanteile in Bezug auf die Fläche des Fluoreszenzbildes auf.

Die Validitätsinformation VI ergibt sich dann beispielsweise gemäß der Vorschrift

$$VI = \begin{cases} 1 & fuer\ (TF1 > SW1) \wedge (TF2 > SW2) \\ 0 & sonst \end{cases}$$

Zusammengefasst kann festgestellt werden, dass unter Verwendung des Schrittes S82 aus der Fig. 3 also das Konfidenzmaß der tatsächlichen Präsenz des bestimmten Fluoreszenzmustertyps in dem Fall ausgegeben wird, dass die jeweiligen Flächenanteile der jeweiligen Flächen TF1, TF2 einen jeweiligen Schwellenwert SW1, SW2 überschreiten. Hierdurch wird insbesondere sichergestellt, dass nicht nur eine der relevanten Teilflächen bzw. eine der relevanten Organschichten zu einem hinreichenden Maß in dem Fluoreszenzbild präsent ist, sondern dass dies für alle relevanten Teilflächen bzw. alle relevanten Organschichten der Fall ist.

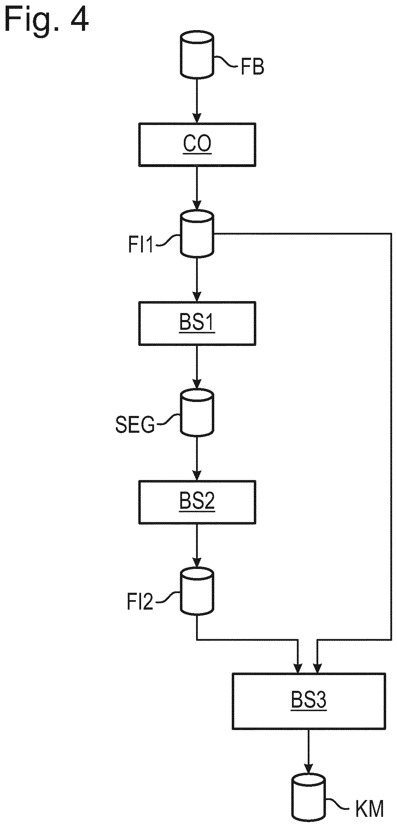

Die Fig. 4 zeigt bevorzugte Schritte zur Ermittlung unterschiedlicher Mengen an Feature-Informationen. Gemäß der Fig. 4 sind Schritte dargestellt, welche in dem neuronalen Netz NN bevorzugt durchgeführt werden können.

In einem Schritt CO erfolgt eine Prozessierung des Fluoreszenzbildes FB mittels einer oder mehrerer Convolutional-Operationen. Es wird dann also in dem Schritt CO auf Basis des Fluoreszenzbildes FB eine erste Menge FI1 mehrerer Feature-Informationen in einem Merkmalsraum mittels der einen oder mehreren Convolutional-Operationen generiert. Anschließend wird später auf Basis dieser Menge FI1 an Feature-Informationen die Segmentierungsinformation SEG und das Konfidenzmaß KM bestimmt.

Im Gegensatz zu Verfahren aus dem Stand der Technik, bei welchen zunächst in einem ersten Netzwerk ein Fluoreszenzbild FB analysiert wird, um eine Segmentierungsinformation SEG zu bestimmen, um dann die Segmentierungsinformation SEG als sogenannte Bildmaske über das Fluoreszenzbild FB zu legen, und dann im Weiteren ein maskiertes Fluoreszenzbild in einem weiteren Netzwerk zu analysieren, um das Konfidenzmaß KM zu bestimmen, wird hiervon explizit abgewichen. Das eine neuronale Netz NN bestimmt gleichzeitig die Segmentierungsinformation SEG als auch das Konfidenzmaß KM. Hierdurch können also in einem Merkmalsraum sowohl Segmentierungsinformation SEG als auch Konfidenzmaß KM gleichzeitig bestimmt werden und diese sich gegenseitig während einer Trainingsphase des neuronalen Netzes NN bedingen. Hierdurch wirkt sich also die Segmentierungsinformation SEG noch im Merkmalsraum auf die Ermittlung des Konfidenzmaßes KM aus.

Nach Generierung der ersten Menge an Feature-Informationen FI1 erfolgt dann in einem Bestimmungsschritt BS1 die Bestimmung der Segmentierungsinformation SEG auf Basis der ersten Menge an Feature-Informationen FI1. Es erfolgt erst dann anschließend eine Bestimmung des Konfidenzmaßes KM auf Basis der ersten Menge an Feature-Informationen FI1 und auf Basis der Segmentierungsinformation SEG. Auch hier bestimmt das neuronale Netz NN also gleichzeitig die Segmentierungsinformation SEG und das Konfidenzmaß KM.

Vorzugsweise wird die Segmentierungsinformation SEG, welche in dem Bestimmungsschritt BS1 auf Basis der Feature-Informationen FI1 bestimmt wurde, in einem Bestimmungsschritt BS2 nochmals in dem Merkmalsraum in eine zweite Menge an Feature-Informationen FI2 transformiert. Es wird also auf Basis der Segmentierungsinformation SEG eine zweite Menge mehrerer Feature-Informationen FI2 in einem Merkmalsraum mittels wenigstens einer Convolutional-Operation generiert. Erst dann wird in dem Merkmalsraum in einem Bestimmungsschritt BS3 auf Basis der ersten Menge an Feature-Informationen FI1 und der zweiten Menge an Feature-Informationen FI2 das Konfidenzmaß KM bestimmt.

Mit anderen Worten: Die Segmentierungsinformation SEG wird also in einem Bestimmungsschritt BS2 in einen Merkmalsraum bzw. eine Feature-Information FI2 transformiert, welche dann in dem Merkmalsraum zusammen mit der ersten Feature-Information FI1 herangezogen wird, um das Konfidenzmaß KM zu bestimmen. Die Segmentierungsinformation SEG wird also nicht, wie aus dem Stand der Technik bekannt, direkt auf das Fluoreszenzbild FB angewendet, sondern in dem Merkmalsraum transformiert als eine Feature-Information FI2 und dort in dem Merkmalsraum findet dann die Segmentierungsinformation SEG Eingang in die Bestimmung des Konfidenzmaßes KM, insbesondere zusammen mit der ersten Menge an Feature-Informationen FI1.

Es wird also zunächst das gesamte Fluoreszenzbild FB in den Merkmalsraum als eine Feature-Information FI1 transformiert und es erfolgt vor der Bestimmung der Segmentierungsinformation SEG keine Rücktransformation in den Bildraum.

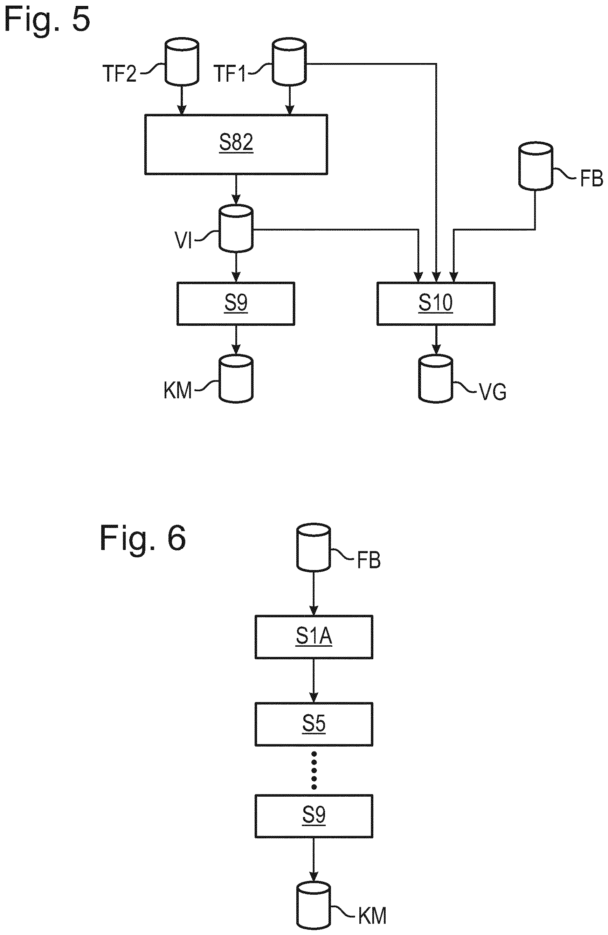

Die Fig. 5 zeigt bevorzugte Schritte zum Abschätzen eines maximalen Verdünnungsgrades der Patientenprobe, bei welchem eine Inkubation des Organschnittes mit der Patientenprobe noch zu einer Präsenz des Fluoreszenzmustertyps führt.

Die Teilflächen TF1 und TF2 werden in einem Schritt S82, wie zuvor in Bezug auf die Fig. 3 beschreiben, herangezogen, um die Validitätsinformation VI zu bestimmen. In dem zuvor in Bezug auf die Fig. 1 erwähnten Schritt S9 kann dann die Ausgabe des Konfidenzmaßes KM in Abhängigkeit der Validitätsinformation VI erfolgen.

Ein weiterer bevorzugter Schritt S10 ist in der Fig. 5 dargestellt. Indiziert die Validitätsinformation VI, dass der Fluoreszenzmustertyp als tatsächlich präsent bestimmt wird, so kann dann in dem Schritt S10 ein Helligkeitsgrad einer Teilfläche in dem Fluoreszenzbild bestimmt werden, wobei die Teilfläche für eine Ausbildung des Fluoreszenzmustertyps potentiell relevant ist. Beispielsweise kann hier in dem Fall, dass an sich zwei Organschichten relevant sind, nur eine einzelne Organschicht herangezogen werden. Hierbei kann es sich beispielsweise um die Organschicht Tunica muscularis handeln, zu welcher in der Fig. 9a eine Teilfäche TF1 dargestellt ist.

In dem Schritt S10 erfolgt dann das Abschätzen des maximalen Verdünnungsgrades als eine Information VG auf Basis der Teilfläche TF1 und des Fluoreszenzbildes FB. Es kann hierbei beispielsweise in einer bevorzugten Ausführungsform die Segmentierungsinformation der Teilfläche TF1, siehe Fig. 9a als Segmentierungsinformation SEG1 als Maske über das Fluoreszenzbild FB gelegt werden und dann der Helligkeitsgrad bzw. Intensitätsgrad in diesem Teilflächenbereich ermittelt werden und auf Basis dieses Helligkeitsgrades der entsprechende maximale Verdünnungsgrad abgeschätzt werden.

Es wird hierfür vorzugsweise eine Pixelstatistik auf diesem relevanten Fluoreszenzbildbereich der Organschicht durchgeführt. Es wird das 95%-Quantil der Helligkeitswerte aus dem Teilbild TF1 des Fluoreszenbildes bestimmt. Die Helligkeitswerte können beispielsweise im Bereich von 0 bis 255 quantisiert sein. Dieser gesamte Quantisierungsbereich der Helligkeitswerte von 0 bis 255 kann dann in fünf Teilwertebereiche äquidistant unterteilt werden. Der erste Bereich reicht dann von 0 bis 51. Die weiteren Bereiche schließen sich in entsprechenden äquidistanten Schritten an, wobei der oberste fünfte Bereich bei 255 endet. Auf Basis des Helligkeitsgrades in Form des 95%-Quantils kann dann eine Abschätzung eines maximalen Verdünnungsgrades der Patientenprobe durchgeführt werden, bei welchem eine Inkubation des Organschnittes mit der Patientenprobe noch zu einer Präsenz eines bzw. des Fluoreszenzmustertyps führt. Die zu bestimmende Information HI als das 95 % Quantil wird dann entsprechend einem der Teilwertbereiche zugeordnet. Der ermittelte Teilwertbereich bzw. der Index des ermittelten Teilwertbereiches bestimmt eine Schrittgröße, ausgehend von der vorliegenden Verdünnung der Patientenprobe für die Erzeugung des Fluoreszenzbildes, um einen Verdünnungsgrad festzulegen, bei welchem die Patientenprobegrade noch zu einem positiven Muster führen würde bzw. zu einer Präsenz des Fluoreszenzmustertyps. Es wird also als Nebeninformation der Verdünnungsgrad VD der Probe aus der Inkubation bereitgestellt. Bei einer Verdünnung bzw. einem Verdünnungsgrad VD von 1 : 10 kann dann bei einer 10er-Verdünnungsreihe der Abstufung

10, 32, 100, 320, 1.000, 3.200, 10.000, 32.000

ausgehend von dem Wert 10 aufgrund einer ermittelten Schrittgröße, beispielsweise 2, und zwei Schritte weiter gegangen werden und dann eine Verdünnung von 100 ermittelt werden als ein Verdünnungsgrad, bei welchem eine Inkubation des Organschnittes mit der Patientenprobe gerade noch zu einer Präsenz des Fluoreszenzmustertyps führen würde. Dies ist dann der ermittelte Verdünnungsgrad VG.

Die Fig. 6 zeigt bevorzugte Schritte einer bevorzugten Ausführungsform eines vorgeschlagenen Verfahrens zur digitalen Bildverarbeitung. In einem Schritt S1A wird das Fluoreszenzbild FB entgegengenommen. An den Schritt S1A schließen sich die Schritte S5 bis S9 aus der Fig. 1 an.

Vorgeschlagen wird ferner ein Computerprogrammprodukt, umfassend Befehle, die bei der Ausführung des Programms durch einen Computer diesen veranlassen, das Verfahren zur digitalen Bildverarbeitung der vorgeschlagenen Form durchzuführen.

Gemäß der Fig. 1 kann das Konfidenzmaß KM in einem weiteren Schritt S9A noch einmal überprüft werden. Weist das Konfidenzmaß KM einen Wert auf, der einen bereitgestellten Schwellenwert SWX übersteigt, so wird dann das Konfidenzmaß KM ausgegeben. Hierbei muss also das Konfidenzmaß KM eine Mindestsicherheit bezüglich der Präsenz des bestimmten Fluoreszenzmustertypen aufweisen.

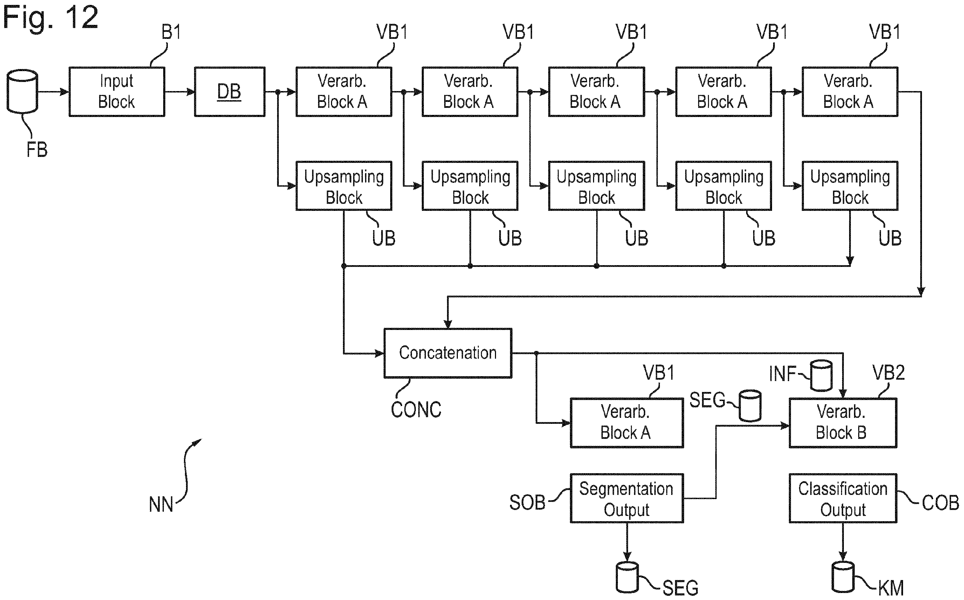

Die Figur 12 zeigt eine Übersichtsstruktur einer bevorzugten Ausführungsform des neuronalen Netzes NN, welches das Fluoreszenzbild FB entgegennimmt. In einem Verarbeitungsblock B1 wird das Fluoreszenzbild FB entgegengenommen und vorverarbeitet. Es folgt ein Block DB, in welchem ein Downsampling durchgeführt wird.

Die Ausgangsgröße des Blocks DB wird dann einem Verarbeitungsblock VB1 und auch einem Upsampling Block UB zugeführt. Es folgen vier weitere Blockanordnungen, bei welchen jeweils die Ausgangsgröße eines Verarbeitungsblocks VB1 einem weiteren folgenden Verarbeitungsblock VB1 als auch einem Upsampling Block UB zugeführt wird.

Die Ausgangsgröße der Upsampling Blöcke UB sowie die Ausgangsgröße des letzten Verarbeitungsblockes VB1 werden in einem Concatenation Block CONC konkateniert.

Die Ausgangsgröße des Concatenation Blockes CONC wird dann einem Verarbeitungsblock VB1 als auch einem Verarbeitungsblock VB2 zugeführt.

Die Ausgangsgröße des Verarbeitungsblockes VB1 wird dann einem Block SOB zur Ausgabe eines Segmentationsergebnisses SEG zugeführt. In dem Block SOB wird dann das Segmentierungsergebnis bzw. die Segmentierungsinformation SEG ausgegeben.

Diese Segmentierungsinformation SEG wird ebenfalls einem weiteren Verarbeitungsblock VB2 zugeführt. Der Verarbeitungsblock VB2 ermittelt aus der Ausgangsgröße INF des Concatenation Blockes CONC und der Segmentierungsinformation SEG dann das Konfidenzmaß KM, welches in einem Block COB ausgegeben wird.

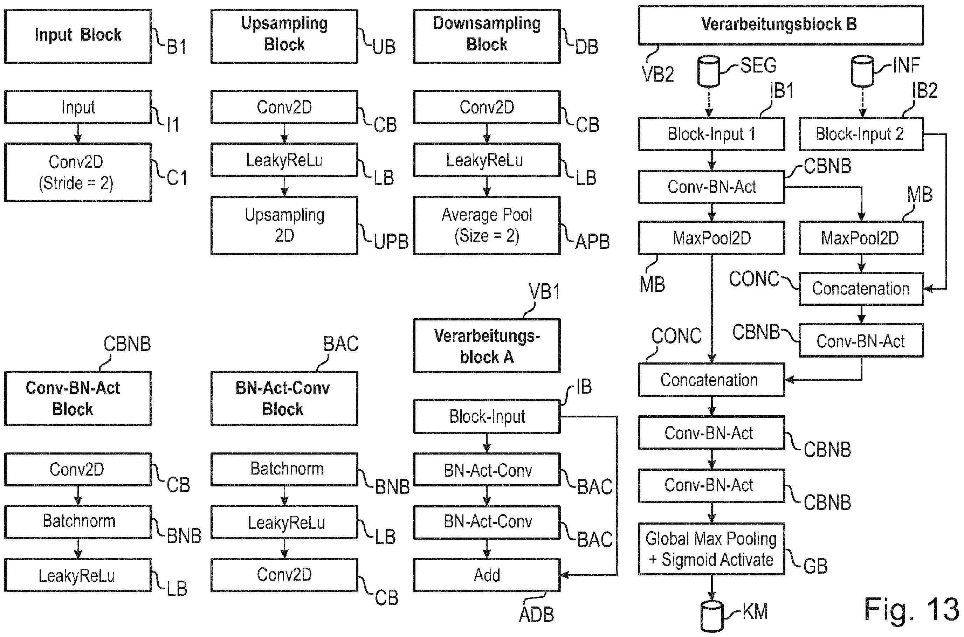

Details zu den dargestellten Blöcken aus der Figur 12 befinden sich in der Figur 13.

Der Eingangsblock B1 weist einen Block I1 auf, in welchem eine Eingangsgröße entgegengenommen wird und einen folgenden Block C1, in welchem eine zweidimensionale Faltung, "2-dimensional convolution", durchgeführt wird. Dies erfolgt vorzugsweise mit einer Größe Stride=2.

Ein Upsampling Block UB weist zunächst einen Block CB auf, in welchem eine zweidimensionale Faltung bzw. eine "2-dimensional convolution" durchgeführt wird. Es folgt ein Block LB mit einer LeakyReLU Funktion. Ferner folgt ein so genannter Upsampling Block UPB.

Ein Downsampling Block DB weist zunächst einen Block CB auf, welchem ein Block LB folgt, auf welchen dann ein Block APB folgt, in welchem ein Average Pooling mit der Größe 2, Size=2, durchgeführt wird.

Ein Verarbeitungsblock VB1 weist zunächst einen Block IB auf, in welchem eine Eingangsgröße entgegengenommen wird und dann unterschiedlichen Blöcken BAC, ADB zugeführt wird. In einem Block BAC erfolgt eine Abfolge der Operation Batchnorm, Activation, Convolution. In einem Block ADB erfolgt ein elementweises Addieren der mehreren dem Block ADB bereitgestellten Eingangsgrößen, hier aus dem Block IB und einem Block BAC.

Ein Convolutional-Batchnorm-Activation Block CBNB weist zunächst einen Block CB, dann einen Batchnorm Block BNB und dann einen Block LB auf.

Ein Batchnorm-Activation-Convolution Block BAC weist zunächst einen Block BNB, dann einen Block LB und dann einen Block CB auf.

Ein Verarbeitungsblock VB2 weist einen Eingangsblock IB1 auf, in welchen die Segmentierungsinformation SEG eingeht.

Ein weiterer, paralleler Eingangsblock IB2 nimmt die Information INF entgegen, welche auch in der Figur 12 dargestellt ist. Die Information INF ist jene Information, welche dort der Concatenation Block CONC generiert und an den Verarbeitungsblock VB2 übergibt.

Die Segmentierungsinformation SEG wird dann nach dem Block IB1 in einen Block CBNB übergeben. Die dort generierte Information wird zum einen an einen linksseitigen Max Pooling 2D Block MB übergeben sowie an einen rechtsseitigen Max Pooling 2D Block MB.

In dem linksseitigen Verarbeitungsstrang wird dann die von dem Block MB ermittelte Größe an einen Concatenation Block CONC übergeben. In dem rechtsseitigen Strang wird eine Abfolge eines Blockes MB, eines Blockes CONC und eines Blocks CBNB durchgeführt, bevor die entsprechend ermittelte Information auch an den Concatenation Block CONC übergeben wird. Es folgen zwei Blöcke vom Typ CBNB.

Schließlich wird in einem Block GB ein Global Max Pooling und eine Sigmoid Aktivierung durchgeführt. Hierdurch wird die Konfidenzmaßinformation KM ermittelt.

Für eine Implementation eines oder mehrerer Ausführungsbeispiele des hier vorgeschlagenen Convolutional Neural Networks NN kann ein Fachmann auf eine sogenannte Open Source Deep-Learning Bibliothek namens "Keras" zurückgegriffen werden. Detaillierte Informationen findet der Fachmann unter https://keras.io .

Ergebnisse

Die Leistungsdaten wurden mit 78 Patientenproben ermittelt. Jede Probe wurde jeweils in 3 Titrationsstufen austitriert und dann für jede Titrationsstufe jeweils ein Substrat bzw. ein Organschnitt inkubiert. Hierbei wurden die inkubierten Substrate mit dem Mikroskoptyp EURO-Pattern Mikroskop 1.5 in Form von Fluoreszenzbildern erfasst. Je Probe ergaben sich also jeweils 3 Ergebnisse der jeweiligen 3 Titrationsstufen. Wurde durch das vorgeschlagene Verfahren für wenigstens eines der 3 Fluoreszenzbilder der bestimmten Probe eine Präsenz des Fluoreszenzmustertyps als positiv detektiert, so wurde auf eine prinzipielle Präsenz des Fluoreszenzmustertyps entschieden.

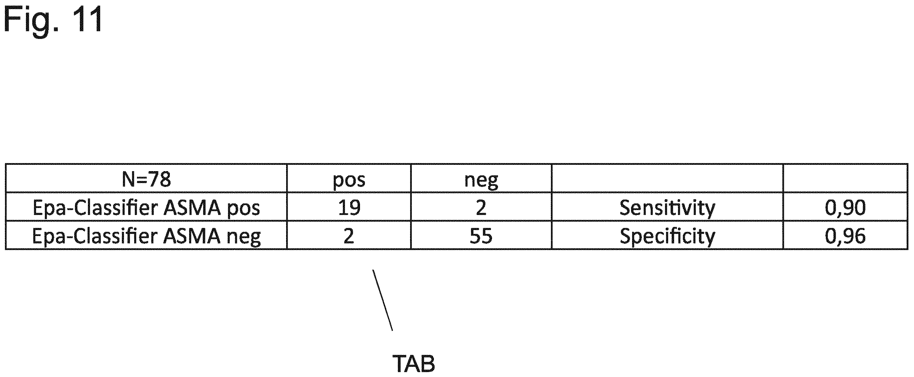

Die Tabelle TAB aus der Figur 11 zeigt hierzu, dass von 21 tatsächlich positiven Proben das vorgeschlagene Verfahren 19 Proben als positive erkannt hat und 2 Proben fälschlicherweise als negativ erkannt wurden. Die Tabelle aus der Figur 11 zeigt ferner, dass von 57 tatsächlich negativen Proben das vorgeschlagene Verfahren 55 Proben als negaitve erkannt hat und 2 Proben fälschlicherweise als positiv erkannt wurden. Hieraus ergibt sich eine analytische Sensitivität von 0,90. Ferner ergibt sich hieraus eine analytische Spezifität von 0,96.

Obwohl manche Aspekte im Zusammenhang mit einer Vorrichtung beschrieben wurden, versteht es sich, dass diese Aspekte auch eine Beschreibung der entsprechenden Verfahren darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung die Recheneinheit R oder die Datennetzwerkvorrichtung in Hardware und/oder in Software umsetzen. Eine Umsetzung einer hier genannten Recheneinheit R kann hier als wenigstens eine Recheneinheit erfolgen oder aber durch mehrere Recheneinheiten im Verbund. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann als Recheneinheit durch einen Prozessor, einen Computerprozessor (CPU = Central Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird.

Allgemein können Ausführungsbeispiele oder Teile der Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren oder ein Teil eines Verfahrens durchzuführen, wenn das Programm auf einem Prozessor oder einer programmierbaren Hardwarekomponente abläuft.

**Patentansprüche**

1. Verfahren zum Detektieren wenigstens einer potentiellen Präsenz wenigstens eines Fluoreszenzmustertyps auf einem Organschnitt mittels Immunfluoreszenzmikroskopie und mittels digitaler Bildverarbeitung, aufweisend

   - Bereitstellen des Organschnittes auf einem Objektträger,
   - Inkubieren des Organschnittes mit einer flüssigen Patientenprobe, welche potentiell primäre Antikörper aufweist,
   - Inkubieren des Organschnittes mit sekundären Antikörpern, welche mit einem Fluoreszenzfarbstoff markiert sind,
   - Erfassen eines Fluoreszenzbildes (FB) des Organschnittes in einem Farbkanal, welcher zu dem Fluoreszenzfarbstoff korrespondiert,
   - Bereitstellen des Fluoreszenzbildes (FB) an ein neuronales Netz (NN),
   - gleichzeitiges Bestimmen einer Segmentierungsinformation (SEG) durch Segmentieren des Fluoreszenzbildes (FB) und ferner eines Konfidenzmaßes (KM), welches eine tatsächliche Präsenz des Fluoreszenzmustertyps indiziert, mittels des einen neuronalen Netzes (NN),
   - Bestimmen wenigstens einer Teilfläche (TF1, TF2) des Fluoreszenzbildes (FB), welche für eine Ausbildung des Fluoreszenzmustertyps relevant ist, auf Basis der Segmentierungsinformation (SEG),
   - Bestimmen einer Validitätsinformation (VI), welche einen Grad einer Validität des Konfidenzmaßes (KM) indiziert, auf Basis der zuvor bestimmten wenigstens einen Teilfläche (TF1, TF2),
   - Ausgeben des Konfidenzmaßes (KM) der tatsächlichen Präsenz des Fluoreszenzmustertyps in Abhängigkeit der Validitätsinformation (VI).

2. Verfahren nach Anspruch 1, ferner aufweisend

   - Bestimmen mehrerer Teilflächen (TF1, TF2) des Fluoreszenzbildes (FB), welche für eine Ausbildung des Fluoreszenzmustertyps relevant sind, auf Basis der Segmentierungsinformation (SEG),
   - Bestimmen der Validitätsinformation (VI), welche einen Grad einer Validität des Konfidenzmaßes (KM) indiziert, auf Basis der zuvor bestimmten Teilflächen (TF1, TF2).

3. Verfahren nach Anspruch 1, ferner aufweisend

   - Bestimmen eines Flächenanteils der wenigstens einen Teilfläche (TF1, TF2) bezogen auf die Fläche des Fluoreszenzbildes (FB) sowie
   - Bestimmen der Validitätsinformation (VI) auf Basis des Flächenanteils.

4. Verfahren nach Anspruch 2, ferner aufweisend

- Bestimmen jeweiliger Flächenanteile der jeweiligen Teilflächen (TF1, TF2) bezogen auf die Fläche des Fluoreszenzbildes (FB) sowie
- Bestimmen der Validitätsinformation (VI) auf Basis der Flächenanteile.

**5.** Verfahren nach Anspruch 2,
ferner aufweisend

- Bestimmen mehrerer Teilflächen (TF1, TF2) des Fluoreszenzbildes (FB), welche für eine Ausbildung des Fluoreszenzmustertyps relevant sind, auf Basis der Segmentierungsinformation (SEG),
- Bestimmen jeweiliger Flächenanteile der jeweiligen Teilflächen (TF1, TF2) bezogen auf die Fläche des Fluoreszenzbildes (FB),
- Bestimmen der Validitätsinformation (VI) auf Basis der Flächenanteile und auf Basis jeweiliger Schwellenwerte,
- Ausgeben des Konfidenzmaßes (KM) der tatsächlichen Präsenz des Fluoreszenzmustertypes in dem Fall, dass die jeweiligen Flächenanteile einen jeweiligen Schwellenwert (SW1, SW2) überschreiten.

**6.** Verfahren nach Anspruch 1,
wobei das neuronale Netz (NN)

- zunächst auf Basis des Fluoreszenzbildes (FB) eine erste Menge (FI1) mehrerer Feature-Informationen in einem Merkmalsraum mittels wenigstens einer oder mehrerer Convolutional-Operationen generiert
- und anschließend auf Basis der ersten Menge (FI1) an Feature-Informationen die Segmentierungsinformation (SEG) und das Konfidenzmaß (KM) bestimmt.

**7.** Verfahren nach Anspruch 1,
wobei das neuronale Netz (NN)

- zunächst auf Basis des Fluoreszenzbildes (FB) eine erste Menge (FI1) mehrerer Feature-Informationen in einem Merkmalsraum mittels einer oder mehrerer Convolutional-Operationen generiert
- ferner auf Basis der ersten Menge (FI1) an Feature-Informationen die Segmentierungsinformation (SEG) bestimmt
- sowie ferner auf Basis der ersten Menge (FI1) an Feature-Informationen und auf Basis der Segmentierungsinformation (SEG) das Konfidenzmaß (KM) bestimmt.

**8.** Verfahren nach Anspruch 1,
wobei das neuronale Netz (NN)

- zunächst auf Basis des Fluoreszenzbildes (FB) eine erste Menge (FI1) mehrerer Feature-Informationen in einem Merkmalsraum mittels einer oder mehrerer Convolutional-Operationen generiert,
- ferner auf Basis der ersten Menge (FI1) an Feature-Informationen die Segmentierungsinformation bestimmt,
- ferner auf Basis der Segmentierungsinformation (SEG) eine zweite Menge (FI2) mehrerer Feature-Informationen in einem Merkmalsraum mittels wenigstens einer Convolutional-Operation generiert
- sowie ferner auf Basis der ersten Menge (FI1) an Feature-Informationen und der zweiten Menge (FI2) an Feature-Informationen das Konfidenzmaß (KM) bestimmt.

**9.** Verfahren nach Anspruch 1,
ferner aufweisend,

- Bestimmen mehrerer Teilflächen (TF1, TF2) des Fluoreszenzbildes (FB), welche für eine Ausbildung des Fluoreszenzmustertyps relevant sind, auf Basis der Segmentierungsinformation (SEG),
- und in dem Fall, dass der Fluoreszenzmustertyp als tatsächlich präsent bestimmt wird, Bestimmen eines Helligkeitsgrades einer der Teilflächen (TF1) in dem Fluoreszenzbild (FB), welche für eine Ausbildung des Fluoreszenzmustertyps potentiell relevant ist,
- sowie Abschätzen eines maximalen Verdünnungsgrades (VG) der Patientenprobe, bei welchem eine Inkubation des Organschnittes mit der Patientenprobe noch zu einer Präsenz eines des Fluoreszenzmustertyps führt.

**10.** Vorrichtung (V1) zum Detektieren wenigstens einer potentiellen Präsenz wenigstens eines Fluoreszenzmustertyps auf einem Organschnitt mittels Immunfluoreszenzmikroskopie und mittels digitaler Bildverarbeitung,
aufweisend

- eine Haltevorrichtung (H) für einen Objektträger mit einem Organschnitt (S), welcher mit einer Patientenprobe, aufweisend die Autoantikörper, sowie ferner mit sekundären Antikörpern, welche jeweils mit einem Fluoreszenzfarbstoff markiert sind, inkubiert wurde,
- wenigstens eine Bilderfassungseinheit (K1, K2) zum Erfassen eines Fluoreszenzbildes (FB) des Organschnittes (S) in einem Farbkanal, welcher zu dem Fluoreszenzfarbstoff korrespondiert,

sowie ferner aufweisend wenigstens eine Recheneinheit (R), welche ausgebildet ist,

- das Fluoreszenzbild (FB) an ein neuronales Netz (NN) bereitzustellen,
- mittels des einen neuronalen Netzes (NN) gleichzeitig eine Segmentierungsinformation (SEG) durch Segmentieren des Fluoreszenzbildes (FB) zu bestimmen und ferner ein Konfidenzmaß (KM), welches eine tatsächliche Präsenz des Fluoreszenzmustertyps indiziert, zu bestimmen,
- wenigstens eine Teilfläche (TF1, TF2) des Fluoreszenzbildes (FB) zu bestimmen, welche für eine Ausbildung des Fluoreszenzmustertyps relevant ist, auf Basis der Segmentierungsinformation (SEG),
- eine Validitätsinformation (VI) zu bestimmen, welche einen Grad einer Validität des Konfidenzmaßes (KM) indiziert, auf Basis der zuvor bestimmten wenigstens einen Teilfläche (TF1, TF2),
- sowie das Konfidenzmaß (KM) der tatsächlichen Präsenz des Fluoreszenzmustertyps in Abhängigkeit der Validitätsinformation (VI) auszugeben.

11. Verfahren zur digitalen Bildverarbeitung
aufweisend

- Entgegennehmen eines Fluoreszenzbildes, welches eine Färbung eines Organschnittes (S), durch einen Fluoreszenzfarbstoff repräsentiert,
- Bereitstellen des Fluoreszenzbildes (FB) an ein neuronales Netz (NN),
- gleichzeitiges Bestimmen einer Segmentierungsinformation (SEG) durch Segmentieren des Fluoreszenzbildes (FB) und Bestimmen eines Konfidenzmaßes (KM), welches eine tatsächliche Präsenz des Fluoreszenzmustertyps indiziert, mittels des einen gemeinsamen neuronalen Netzes (NN),
- Bestimmen wenigstens einer Teilfläche (TF1, TF2) des Fluoreszenzbildes (FB), welche für eine Ausbildung des Fluoreszenzmustertyps relevant ist, auf Basis der Segmentierungsinformation (SEG),
- Bestimmen einer Validitätsinformation (VI), welche einen Grad einer Validität des Konfidenzmaßes (KM) indiziert, auf Basis der zuvor bestimmten wenigstens einen Teilfläche (TF1, TF2),
- Ausgeben des Konfidenzmaßes (KM) der tatsächlichen Präsenz des Fluoreszenzmustertypes in Abhängigkeit der Validitätsinformation (VI).

12. Computerprogrammprodukt, umfassend Befehle, die bei der Ausführung des Programms durch einen Computer diesen veranlassen, das Verfahren zur digitalen Bildverarbeitung nach Anspruch 11 durchzuführen.

**Claims**

1. Method for detecting at least one potential presence of at least one fluorescence pattern type on an organ segment via immunofluorescence microscopy and via digital image processing,
comprising

- providing the organ segment on a slide,
- incubating the organ segment with a liquid patient sample which potentially comprises primary antibodies,
- incubating the organ segment with secondary antibodies which have been labelled with a fluorescent dye,
- acquiring a fluorescence image (FB) of the organ segment in a colour channel corresponding to the fluorescent dye,
- providing the fluorescence image (FB) to a neural network (NN),
- simultaneously determining, by means of the one neural network (NN), segmentation information (SEG) through segmentation of the fluorescence image (FB) and, furthermore, a measure of confidence (KM) indicating an actual presence of the fluorescence pattern type,
- determining, on the basis of the segmentation information (SEG), at least one sub-area (TF1, TF2) of the fluorescence image (FB) that is relevant to formation of the fluorescence pattern type,
- determining, on the basis of the previously determined at least one sub-area (TF1, TF2), validity information (VI) indicating a degree of a validity of the measure of confidence (KM),

- outputting the measure of confidence (KM) of the actual presence of the fluorescence pattern type depending on the validity information (VI).

2. Method according to Claim 1,
   further comprising

   - determining, on the basis of the segmentation information (SEG), multiple sub-areas (TF1, TF2) of the fluorescence image (FB) that are relevant to formation of the fluorescence pattern type,
   - determining, on the basis of the previously determined sub-areas (TF1, TF2), the validity information (VI) indicating a degree of a validity of the measure of confidence (KM).

3. Method according to Claim 1,
   further comprising

   - determining an area fraction of the at least one sub-area (TF1, TF2) based on the area of the fluorescence image (FB) and
   - determining the validity information (VI) on the basis of the area fraction.

4. Method according to Claim 2,
   further comprising

   - determining respective area fractions of the respective sub-areas (TF1, TF2) based on the area of the fluorescence image (FB) and
   - determining the validity information (VI) on the basis of the area fractions.

5. Method according to Claim 2,
   further comprising

   - determining, on the basis of the segmentation information (SEG), multiple sub-areas (TF1, TF2) of the fluorescence image (FB) that are relevant to formation of the fluorescence pattern type,
   - determining respective area fractions of the respective sub-areas (TF1, TF2) based on the area of the fluorescence image (FB),
   - determining the validity information (VI) on the basis of the area fractions and on the basis of respective threshold values,
   - outputting the measure of confidence (KM) of the actual presence of the fluorescence pattern type if the respective area fractions exceed a respective threshold value (SW1, SW2).

6. Method according to Claim 1,
   wherein the neural network (NN)

   - first generates, on the basis of the fluorescence image (FB), a first set (FI1) of a plurality of feature information in a feature space by means of at least one or more convolutional operations
   - and then determines, on the basis of the first set (FI1) of feature information, the segmentation information (SEG) and the measure of confidence (KM).

7. Method according to Claim 1,
   wherein the neural network (NN)

   - first generates, on the basis of the fluorescence image (FB), a first set (FI1) of a plurality of feature information in a feature space by means of one or more convolutional operations
   - furthermore determines, on the basis of the first set (FI1) of feature information, the segmentation information (SEG)
   - and furthermore determines, on the basis of the first set (FI1) of feature information and on the basis of the segmentation information (SEG), the measure of confidence (KM).

8. Method according to Claim 1,
   wherein the neural network (NN)

- first generates, on the basis of the fluorescence image (FB), a first set (FI1) of a plurality of feature information in a feature space by means of one or more convolutional operations,
- furthermore determines, on the basis of the first set (FI1) of feature information, the segmentation information,
- furthermore generates, on the basis of the segmentation information (SEG), a second set (FI2) of a plurality of feature information in a feature space by means of at least one convolutional operation
- and furthermore determines, on the basis of the first set (FI1) of feature information and the second set (FI2) of feature information, the measure of confidence (KM).

**9.** Method according to Claim 1,
further comprising

- determining, on the basis of the segmentation information (SEG), multiple sub-areas (TF1, TF2) of the fluorescence image (FB) that are relevant to formation of the fluorescence pattern type,
- and, in the event of the fluorescence pattern type being determined as actually present, determining a degree of brightness of one of the sub-areas (TF1) in the fluorescence image (FB) that is potentially relevant to formation of the fluorescence pattern type,
- and estimating a maximum degree of dilution (VG) of the patient sample at which incubation of the organ segment with the patient sample still leads to a presence of a fluorescence pattern type or the fluorescence pattern type.

**10.** Device (V1) for detecting at least one potential presence of at least one fluorescence pattern type on an organ segment via immunofluorescence microscopy and via digital image processing,
comprising

- a holding device (H) for a slide containing an organ segment (S) which has been incubated with a patient sample potentially comprising primary antibodies and furthermore with secondary antibodies which have each been labelled with a fluorescent dye,
- at least one image acquisition unit (K1, K2) for acquiring a fluorescence image (FB) of the organ segment (S) in a colour channel corresponding to the fluorescent dye,

and further comprising at least one computing unit (R) designed

- to provide the fluorescence image (FB) to a neural network (NN),
- to simultaneously determine, by means of the one neural network (NN), segmentation information (SEG) through segmentation of the fluorescence image (FB) and, furthermore, a measure of confidence (KM) indicating an actual presence of the fluorescence pattern type,
- to determine, on the basis of the segmentation information (SEG), at least one sub-area (TF1, TF2) of the fluorescence image (FB) that is relevant to formation of the fluorescence pattern type,
- to determine, on the basis of the previously determined at least one sub-area (TF1, TF2), validity information (VI) indicating a degree of a validity of the measure of confidence (KM),
- and to output the measure of confidence (KM) of the actual presence of the fluorescence pattern type depending on the validity information (VI).

**11.** Method for digital image processing,
comprising

- receiving a fluorescence image representing staining of an organ segment (S) due to a fluorescent dye,
- providing the fluorescence image (FB) to a neural network (NN),
- simultaneously determining, by means of the one common neural network (NN), segmentation information (SEG) through segmentation of the fluorescence image (FB) and a measure of confidence (KM) indicating an actual presence of the fluorescence pattern type,
- determining, on the basis of the segmentation information (SEG), at least one sub-area (TF1, TF2) of the fluorescence image (FB) that is relevant to formation of the fluorescence pattern type,
- determining, on the basis of the previously determined at least one sub-area (TF1, TF2), validity information (VI) indicating a degree of a validity of the measure of confidence (KM),
- outputting the measure of confidence (KM) of the actual presence of the fluorescence pattern type depending on the validity information (VI).

12. Computer program product comprising commands which, upon execution of the program by a computer, prompt said computer to carry out the method for digital image processing according to Claim 11.

**Revendications**

1. Procédé de détection d'au moins une présence potentielle d'au moins un type de motif de fluorescence sur une coupe d'organe par microscopie par immunofluorescence et traitement d'image numérique, ledit procédé comprenant les étapes suivantes

   - fournir la coupe d'organe sur un porte-objet,
   - laisser incuber la coupe d'organe avec un échantillon de patient liquide qui contient potentiellement des anticorps primaires,
   - laisser incuber la coupe d'organe avec des anticorps secondaires marqués avec un colorant fluorescent,
   - acquérir une image de fluorescence (FB) de la coupe d'organe dans un canal de couleurs qui correspond au colorant fluorescent,
   - fournir l'image de fluorescence (FB) à un réseau de neurones (NN),
   - déterminer en même temps, au moyen d'un réseau de neurones (NN), une information de segmentation (SEG) par segmentation de l'image de fluorescence (FB) et aussi un niveau de confiance (KM) qui indique une présence réelle du type de motif de fluorescence,
   - déterminer au moins une partie de surface (TF1, TF2) de l'image de fluorescence (FB) qui est pertinente pour former le type de motif de fluorescence sur la base de l'information de segmentation (SEG),
   - déterminer l'information de validité (VI), qui indique un degré de validité du niveau de confiance (KM), sur la base d'au moins une partie de surface préalablement déterminée (TF1, TF2),
   - délivrer en sortie le niveau de confiance (KM) de la présence réelle du type de motif de fluorescence en fonction de l'information de validité (VI).

2. Procédé selon la revendication 1,
   comportant en outre les étapes suivantes

   - déterminer plusieurs parties de surface (TF1, TF2) de l'image de fluorescence (FB) qui sont pertinentes pour former le type de motif de fluorescence sur la base de l'information de segmentation (SEG),
   - déterminer l'information de validité (VI), qui indique un degré de validité du niveau de confiance (KM), sur la base des parties de surface préalablement déterminées (TF1, TF2).

3. Procédé selon la revendication 1,
   comportant en outre les étapes suivantes

   - déterminer une proportion de surface de l'au moins une partie de surface (TF1, TF2) par rapport à la surface de l'image de fluorescence (FB) et
   - déterminer l'information de validité (VI) sur la base de la proportion de surface.

4. Procédé selon la revendication 2,
   comportant en outre les étapes suivantes

   - déterminer les proportions de surface respectives des parties de surfaces respectives (TF1, TF2) par rapport à la surface de l'image de fluorescence (FB) et
   - déterminer l'information de validité (VI) sur la base des proportions de surface.

5. Procédé selon la revendication 2,
   comportant en outre les étapes suivantes

   - déterminer plusieurs parties de surface (TF1, TF2) de l'image de fluorescence (FB), qui sont pertinentes pour former le type de motif de fluorescence, sur la base de l'information de segmentation (SEG),
   - déterminer les proportions de surface respectives des parties de surface respectives (TF1, TF2) par rapport à la surface de l'image de fluorescence (FB),
   - déterminer l'information de validité (VI) sur la base des proportions de surface et sur la base de valeurs seuils respectives,

- délivrer en sortie le niveau de confiance (KM) de la présence réelle du type de motif de fluorescence dans le cas où les proportions de surface respectives dépassent une valeur seuil respective (SW1, SW2).

6. Procédé selon la revendication 1,
le réseau de neurones (NN)

- générant tout d'abord, sur la base de l'image de fluorescence (FB), un premier ensemble (FI1) de plusieurs informations de caractéristiques dans un espace de caractéristiques au moyen d'au moins une ou plusieurs opérations de convolution,
- déterminant ensuite, sur la base du premier ensemble (FI1) d'informations de caractéristiques, les informations de segmentation (SEG) et le niveau de confiance (KM).

7. Procédé selon la revendication 1,
le réseau de neurones (NN)

- générant tout d'abord, sur la base de l'image de fluorescence (FB), un premier ensemble (FI1) de plusieurs informations de caractéristiques dans un espace de caractéristiques au moyen d'une ou plusieurs opérations de convolution,
- déterminant en outre, sur la base du premier ensemble (FI1) d'informations de caractéristiques, l'information de segmentation (SEG),
- et déterminant également, sur la base du premier ensemble (FI1) d'informations de caractéristiques et sur la base des informations de segmentation (SEG), le niveau de confiance (KM).

8. Procédé selon la revendication 1,
le réseau de neurones (NN)

- générant tut d'abord, sur la base de l'image de fluorescence (FB), un premier ensemble (FI1) de plusieurs informations de caractéristiques dans un espace de caractéristiques au moyen d'une ou plusieurs opérations de convolution,
- déterminant en outre, sur la base du premier ensemble (FI1) d'informations de caractéristiques, les informations de segmentation,
- générant en outre, sur la base des informations de segmentation (SEG), un deuxième ensemble (FI2) de plusieurs informations de caractéristiques dans un espace de caractéristiques au moyen d'au moins une opération de convolution,
- et déterminant en outre, sur la base du premier ensemble (FI1) d'informations de caractéristiques et du deuxième ensemble (FI2) d'informations de caractéristiques, le niveau de confiance (KM).

9. Procédé selon la revendication 1,
comportant en outre les étapes suivantes

- déterminer plusieurs parties de surface (TF1, TF2) de l'image de fluorescence (FB), qui sont pertinentes pour former le type de motif de fluorescence, sur la base de l'information de segmentation (SEG),
- et dans le cas où le type de motif de fluorescence est déterminé comme étant réellement présent, déterminer un degré de luminosité de l'une des parties de surface (TF1) dans l'image de fluorescence (FB) qui est potentiellement pertinente pour la formation du type de motif de fluorescence,
- et estimer un degré de dilution maximal (VG) de l'échantillon de patient pour lequel l'incubation de la coupe d'organe avec l'échantillon de patient conduit toujours à la présence d'un type de motif de fluorescence.

10. Dispositif (V1) de détection d'au moins une présence potentielle d'au moins un type de motif de fluorescence sur une coupe d'organe par microscopie par immunofluorescence et traitement numérique d'images,
ledit dispositif comportant

- un dispositif de retenue (H) destiné à un porte-objet pourvu d'une coupe d'organe (S), qui a été incubée avec un échantillon de patient contenant les auto-anticorps, et également d'anticorps secondaires qui sont chacun marqués avec un colorant fluorescent,
- au moins une unité d'acquisition d'images (K1, K2) destinée à acquérir une image de fluorescence (FB) de la coupe d'organe (S) dans un canal de couleurs qui correspond au colorant fluorescent,
et comportant également au moins une unité informatique (R) qui est conçue pour

- fournir l'image de fluorescence (FB) à un réseau de neurones (NN),
- déterminer au moyen du réseau de neurones (NN) en même temps une information de segmentation (SEG) par segmentation de l'image de fluorescence (FB) et également déterminer un niveau de confiance (KM) qui indique une présence réelle du type de motif de fluorescence,
- déterminer au moins une partie de surface (TF1, TF2) de l'image de fluorescence (FB), qui est pertinente pour former le type motif de fluorescence, sur la base de l'information de segmentation (SEG),
- déterminer une information de validité (VI), qui indique un degré de validité du niveau de confiance (KM), sur la base d'au moins une partie de surface préalablement déterminée (TF1, TF2),
- et délivrer en sortir le niveau de confiance (KM) de la présence réelle du type de motif de fluorescence en fonction de l'information de validité (VI).

**11.** Procédé de traitement d'image numérique,
ledit procédé comportant les étapes suivantes

- recevoir une image de fluorescence qui représente une coloration d'une coupe d'organe (S) par un colorant fluorescent,
- fournir l'image de fluorescence (FB) à un réseau de neurones (NN),
- déterminer une information de segmentation (SEG) par segmentation de l'image de fluorescence (FB) et en même temps déterminer un niveau de confiance (KM), qui indique une présence réelle du type de motif de fluorescence, au moyen d'un réseau de neurones commun (NN),
- déterminer au moins une partie de surface (TF1, TF2) de l'image de fluorescence (FB), qui est pertinente pour former le type de motif de fluorescence, sur la base de l'information de segmentation (SEG),
- déterminer l'information de validité (VI), qui indique un degré de validité du niveau de confiance (KM), sur la base d'au moins une partie de surface préalablement déterminée (TF1, TF2),
- délivrer en sortie le niveau de confiance (KM) de la présence réelle du type de motif de fluorescence en fonction de l'information de validité (VI).

**12.** Progiciel comprenant des instructions qui, lorsque le programme est exécuté par un ordinateur, ordonnent à celui-ci de mettre en oeuvre le procédé de traitement d'images numériques selon la revendication 11.

Fig. 1

Fig. 2

SEG

S71

TF

TF2 — TF1

S81

VI

Fig. 3

TF1

TF2

S82

SW1

SW2

Fig. 4

FB

CO

FI1

BS1

SEG

BS2

FI2

BS3

KM

# Fig. 5

# Fig. 6

# Fig. 7

Fig. 8

SC5 — Vessel (Gefäß)
SC4 — Muscularis mucosae
SC3 — Submucosa
SC1 — Tunica muscularis (Ring- und Längsmuskel)
SC2 — Tunica mucosa (Schleimhaut mit Parietalzellen und interglandulären Fibrillen)

Fig. 9

a)        b)        c)        d)

SEG1      SEG2      SEG3      SEG4

e)        f)        g)        h)

SEG5      SEG6      SEG7      FB

Fig. 10

a)

b)

# Fig. 11

| N=78 | pos | neg | | |
|---|---|---|---|---|
| Epa-Classifier ASMA pos | 19 | 2 | Sensitivity | 0,90 |
| Epa-Classifier ASMA neg | 2 | 55 | Specificity | 0,96 |

TAB

# Fig. 12

EP 4 016 082 B1

Fig. 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3258247 A1
- EP 3712618 A1

- EP 3767587 A1